# EUROPÄISCHE PATENTSCHRIFT

(11) **EP 3 174 560 B1**
(45) Veröffentlichungstag und Bekanntmachung des Hinweises auf die Patenterteilung: **04.07.2018**
(21) Anmeldenummer: 15753890.1
(22) Anmeldetag: 29.07.2015
(51) Int. Cl.: A61L 2/07, A61J 9/00

(54) **VORRICHTUNG ZUM ERWÄRMEN BZW. STERILISIEREN EINES BABYARTIKELS**
DEVICE FOR HEATING AND STERILIZING A BABY ITEM
DISPOSITIF POUR CHAUFFER OU STÉRILISER UN ARTICLE DE PUÉRICULTURE

(30) Priorität: 29.07.2014 AT 505332014
(43) Veröffentlichungstag der Anmeldung: 07.06.2017
(73) Patentinhaber: Mam Babyartikel Gesellschaft m.b.H., 1160 Wien (AT)
(72) Erfinder: RÖHRIG, Peter, A-1160 Wien (AT)
(74) Vertreter: Sonn & Partner Patentanwälte
(86) Internationale Anmeldenummer: PCT/AT2015/050185
(87) Internationale Veröffentlichungsnummer: WO 2016/015079

(56) Entgegenhaltungen:
- EP-A1- 0 658 349
- WO-A1-00/54818
- WO-A1-97/47224
- US-A- 2 501 193
- US-A- 4 544 529
- US-A- 5 213 776

## Beschreibung

Die Erfindung betrifft eine Vorrichtung zum Erwärmen bzw. Sterilisieren eines Babyartikels, insbesondere einer Babyflasche, mit einer Dampferzeugungseinheit, die mit einer wannenförmigen Wasseraufnahmeeinheit verbunden ist, wobei die Wasseraufnahmeeinheit in einer ersten Funktionsstellung mit einer Trageinheit mit einer Aufstandsfläche für den Babyartikel verbunden ist.

Aus dem Stand der Technik sind bereits verschiedenste Vorrichtungen zum Desinfizieren bzw. zum Sterilisieren von Babyartikeln wie Flaschenkörpern, insbesondere von Babyflaschen sowie dem zugehörigen Flaschensauger bekannt. Darüber hinaus sind bereits Vorrichtungen bekannt geworden, welche wahlweise zum Erwärmen eines mit Babynahrung befüllten Behälters, wie einer Babyflasche, oder zum Sterilisieren eines leeren Behälters verwendbar sind.

Aus der US 4,544,529 ist bereits eine Vorrichtung der eingangs angeführten Art zum Sterilisieren eines Babyartikels bekannt.

Aus der US 2,501,193 ist beispielsweise eine solche Vorrichtung bekannt, welche einen Wasseraufnahmebehälter und einen damit verbundenen Erhitzer aufweist. Auf den Wasseraufnahmebehälter ist ein Basisteil aufgesetzt, welcher einen Gehäuseteil zur Aufnahme einer Babyflasche stützt. In dem Basisteil sind Öffnungen zum Durchtritt von Wasserdampf aus dem Wasseraufnahmebehälter in den Gehäuseteil ausgebildet. Die Babyflasche kann einerseits in einer aufrechten Stellung in dem Gehäuse angeordnet werden, um den Inhalt einer gefüllten Babyflasche zu erwärmen. Zur Sterilisation einer leeren Babyflasche weist die Vorrichtung einen hülsenförmigen Adapter auf, welcher in dem Gehäuse aufnehmbar ist. Die Babyflasche wird verkehrt herum in dem Gehäuse angeordnet, wobei der Wasserdampf zunächst durch den Adapter in das Innere der Babyflasche und danach entlang der Außenseite der Babyflasche nach außen geführt wird.

Diese Vorrichtung weist jedoch den Nachteil auf, dass für die Erwärmfunktion und für die Sterilisationsfunktion dasselbe Gehäuse zur Aufnahme der Babyflasche verwendet wird. Dadurch muss die Babyflasche bei der Sterilisation in einer aufrechten, kopfstehenden Lage angeordnet werden, welche nachteiligerweise viel Raum einnimmt. Aus diesem Grund wäre es beim Stand der Technik beispielsweise nicht ohne weiteres möglich, die Vorrichtung ohne den Erhitzer in einer Mikrowelle zu verwenden. Darüber hinaus erfordert die Vorrichtung gemäß der US 2,501,193 eine vergleichsweise umständliche Handhabung des Adapters, um die Sterilisationsfunktion der Vorrichtung nutzen zu können.

Demgegenüber besteht die Aufgabe der vorliegenden Erfindung darin, die Nachteile des Standes der Technik zu lindern bzw. zu eliminieren. Die Erfindung setzt sich daher insbesondere zum Ziel, eine konstruktiv einfache Vorrichtung der eingangs angeführten Art zu schaffen, wobei mit geringem baulichen Aufwand eine variable Verwendbarkeit für unterschiedliche Einsatzzwecke erzielbar sein soll. Darüber hinaus soll ein Sterilisationsbehälter und eine Abdeckplatte für eine solche Vorrichtung geschaffen werden.

Diese Aufgabe wird durch eine Vorrichtung gemäß Anspruch 1 gelöst. Bevorzugte Ausführungen sind in den abhängigen Ansprüchen angegeben.

Erfindungsgemäß ist daher die Wasseraufnahmeeinheit alternativ mit einer Trageinheit, welche eine Aufstandsfläche zum Aufstellen eines Babyartikels aufweist, und mit einer Abdeckplatte, welche einen rohrförmigen Dampfauslass zum Austritt von Wasserdampf aufweist, verbindbar. Die Trageinheit und die Abdeckplatte sind als modulare Aufsatzelemente ausgebildet, welche vom Benutzer je nach Verwendungszweck an der Wasseraufnahmeeinheit anordenbar sind. Mit anderen Worten weist die erfindungsgemäße Vorrichtung somit zumindest zwei Module auf, welche mit der Wasseraufnahmeeinheit (dampfleitend) verbindbar sind, nämlich ein Trag-Modul und ein Abdeck-Modul mit einem rohrförmigen Dampfauslass. Die Trageinheit ist insbesondere zur stehenden Anordnung einer Babyflasche eingerichtet, um den Inhalt der Babyflasche mittels Wasserdampf aus der wannenförmigen Wasseraufnahmeeinheit zu erwärmen. Zu diesem Zweck weist die Trageinheit bevorzugt zumindest eine Durchtrittsöffnung auf, mit welcher in der ersten Funktionsstellung Wasserdampf aus der Wasseraufnahmeeinheit zu einer den Babyartikel aufnehmenden Aufnahmekammer der Trageinheit förderbar ist. Durch Beaufschlagung der Babyflasche mit dem Wasserdampf kann der Inhalt der Babyflasche erwärmt werden. In der ersten Funktionsstellung ist die wannenförmige Wasseraufnahmeeinheit bevorzugt derart mit der Trageinheit verbunden, dass im Wesentlichen der gesamte Wasserdampf aus der Wasseraufnahmeeinheit durch die Durchtrittsöffnungen in die Aufnahmekammer der Trageinheit führbar ist. Zur Erzeugung des Wasserdampfes ist die Wasseraufnahmeeinheit mit der Dampferzeugungseinheit verbunden, welche insbesondere elektrische Heizmittel aufweist, um das Wasser in der Wasseraufnahmeeinheit zu verdampfen. Die wannenförmige Wasseraufnahmeeinheit kann, wie erwähnt, alternativ mit der Abdeckplatte versehen werden, wobei der Wasserdampf über den rohrförmigen Dampfauslass aus der Dampfkammer zwischen der Wasseraufnahmeeinheit und der Abdeckplatte abführbar ist. Wenn die Wasseraufnahmeeinheit in der zweiten Funktionsstellung mit der Abdeckplatte verbunden ist, wird daher an dem rohrförmigen Dampfauslass heißer Wasserdampf zur Verfügung gestellt, welcher vorzugsweise einem Sterilisationsbehälter zum Sterilisieren bzw. Desinfizieren eines darin befindlichen Babyartikels zugeführt wird. Die Modulbauweise der erfindungsgemäßen Vorrichtung bringt den Vorteil mit sich, dass dieselbe Wasseraufnahmeeinheit für verschiedene Funktionen verwendet werden kann. Dadurch wird eine teilesparende und kostengünstige Vorrichtung geschaffen, welche sich zudem durch geringen Platzbedarf in der Aufbewahrung auszeichnet.

Um eine Erhitzung des Babyartikels, insbesondere zu Zwecken der Desinfektion oder Sterilisation, durchzuführen, ist erfindungsgemäß vorgesehen, dass die Abdeckplatte in der zweiten Funktionsstellung mit einem Sterilisationsbehälter zur Aufnahme des Babyartikels verbunden ist, wobei der rohrförmige Dampfauslass der Abdeckplatte mit einem Dampfeintrittsventil des Sterilisationsbehälters in Verbindung steht. Demnach gelangt in der zweiten Funktionsstellung Wasserdampf aus der Wasseraufnahmeeinheit über den rohrförmigen
Dampfauslass in das Dampfeintrittsventil des Sterilisationsbehälters, um eine den Babyartikel aufnehmende Aufnahmekammer des Sterilisationsbehälters mit dem Wasserdampf zu versorgen. Durch den Kontakt mit dem heißen Wasserdampf wird der Babyartikel erhitzt. Bei dieser Ausführung kann daher dem Sterilisationsbehälter auf besonders einfache Weise der Wasserdampf zugeführt werden, welcher mittels der Dampferzeugungseinheit in der Wasseraufnahmeeinheit erzeugt wird. Der Sterilisationsbehälter ist lösbar mit der Abdeckplatte verbunden, so dass der Sterilisationsbehälter nach Gebrauch von der Abdeckplatte abgenommen werden kann. Vorteilhafterweise kann daher der Sterilisationsbehälter in einer weiteren Funktionsstellung ohne die Wasseraufnahmeeinheit und die Abdeckplatte verwendet werden.

Um den Dampfeintritt in den Sterilisationsbehälter selektiv absperren zu können, ist es günstig, wenn das Dampfeintrittsventil des Sterilisationsbehälters mittels des rohrförmigen Dampfauslasses der Abdeckplatte aus einer eine Dampfeintrittsöffnung verschließenden Schließstellung in eine die Dampfeintrittsöffnung freigebenden Offenstellung überführbar ist. Demnach ist der rohrförmige Dampfauslass derart ausgestaltet, dass das Dampfeintrittsventil beim Verbinden des Sterilisationsbehälters mit der Abdeckplatte in die Offenstellung gebracht wird, um einen Dampfdurchtritt zwischen der Wasseraufnahmeeinheit und dem Sterilisationsbehälter zu ermöglichen. Andererseits kann das Dampfeintrittsventil in die Schließstellung überführt werden, um bei einer Verwendung des Sterilisationsbehälters ohne die Wasseraufnahmeeinheit, die Abdeckplatte und die Dampferzeugungseinheit einen Wasser- bzw. Dampfaustritt durch das Dampfeintrittsventil zu verhindern. Demnach kann der Sterilisationsbehälter in der Schließstellung des Dampfeintrittsventils mit Wasser befüllt werden, welches bei einer Verwendung des Sterilisationsbehälters in der Mikrowelle verdampft wird. Dadurch kann der in dem Sterilisationsbehälter aufgenommene Babyartikel zuverlässig sterilisiert (oder zumindest desinfiziert) werden. Vorteilhafterweise kann daher der Sterilisationsbehälter auch für sich genommen verwendet werden, um eine Erhitzung des Babyartikels in einer Mikrowelle durchzuführen. Darüber hinaus ist auch eine Verwendung des Sterilisationsbehälters für eine sogenannte Kaltsterilisation möglich, wobei mit speziellen Sterilisationstabletten eine Wasserlösung vorbereitet wird, um die Babyartikel in dem Sterilisationsbehälter ohne Erwärmung von Keimen zu befreien.

Um das Öffnen des Dampfeintrittsventils mittels des rohrförmigen Dampfauslasses mit konstruktiv einfachen Mittel zu bewerkstelligen, ist es vorteilhaft, wenn das Dampfeintrittsventil des Sterilisationsbehälters einen an einem stutzenförmigen Ventilsitz verschieblich gelagerten Ventilkörper aufweist, wobei der Ventilköper durch die Anordnung des rohrförmigen Dampfauslasses der Abdeckplatte in der Offenstellung angeordnet ist. Der Ventilkörper ist bevorzugt hülsenförmig ausgebildet, wobei zumindest eine Dampfeintrittsöffnung an der Mantelfläche des hülsenförmigen Ventilkörpers ausgebildet ist. Beim Verbinden des Sterilisationsbehälters mit der Abdeckplatte wird der Ventilkörper durch den rohrförmigen Dampfauslass selbsttätig in die Offenstellung geschoben, so dass ein Dampffluss zwischen der Wasseraufnahmeeinheit und der Aufnahmekammer des Sterilisationsbehälters freigegeben ist. Wenn der Sterilisationsbehälter von der Wasseraufnahmeeinheit abgenommen ist, kann das Dampfeintrittsventil durch Verschieben des Ventilkörpers in die Schließstellung gebracht werden. In der Schließstellung des Dampfeintrittsventils kann der Sterilisationsbehälter ohne die Wasseraufnahmeeinheit bzw. die Dampferzeugungseinheit für eine Erhitzung des Babyartikels in einer Mikrowelle verwendet werden. Hierbei ist es günstig, wenn der stutzenförmige Ventilsitz des Dampfeintrittsventils von einem Bodenteil des Sterilisationsbehälters nach innen in den Aufnahmeraum vorspringt. Dadurch kann eine bestimmte Wassermenge in den Aufnahmeraum des Sterilisationsbehälters eingefüllt werden, ohne dass das Wasser durch das Dampfeintrittsventil austreten kann.

Um einen Druckaufbau in dem Dampfeintrittsventil zu verhindern, ist bevorzugt vorgesehen, dass die zumindest eine Dampfeintrittsöffnung des Dampfeintrittsventils gleich groß oder größer als der Austrittsquerschnitt des Dampfauslasses der Abdeckplatte ist.

Um das Dampfeintrittsventil selbsttätig, ohne Zutun des Benutzers in die Offenstellung zu bringen, wenn der Sterilisationsbehälter mit der Abdeckplatte verbunden wird, weist der Ventilkörper des Dampfeintrittsventils bevorzugt an der Unterseite eine insbesondere ringförmig umlaufende Anschlagfläche auf, welche mit einer entsprechenden Anschlagfläche an der Oberseite des rohrförmigen Dampfauslasses zusammenwirkt. Beim Verbinden des Sterilisationsbehälters mit der Abdeckplatte wird der Ventilkörper über die Anschlagflächen zwischen dem Ventilkörper und dem rohrförmigen Dampfauslass in die Offenstellung verschoben. Vorteilhafterweise muss daher das Dampfeintrittsventil nicht manuell geöffnet werden, so dass der Dampftransfer zwischen der mit der Abdeckplatte versehenen Wasseraufnahmeeinheit und dem Sterilisationsbehälter im verbundenen Zustand stets freigegeben ist.

Um die Verbindung des Sterilisationsbehälters mit der Abdeckplatte zu erleichtern, ist es günstig, wenn der stutzenförmige Ventilsitz des Dampfeintrittsventils im Wesentlichen senkrecht von einem Bodenteil des Sterilisationsbehälters nach innen vorspringt, wobei der rohrförmige Dampfauslass im Wesentlichen senkrecht von der Hauptebene der Abdeckplatte hochsteht. Demnach kann der Sterilisationsbehälter auf die Abdeckplatte aufgesetzt werden, wobei der rohrförmige Dampfauslass der Abdeckplatte das Dampfeintrittsventil in die Offenstellung verschiebt.

Um für einen zweckmäßigen Dampftransfer zwischen der Wasseraufnahmeeinheit und dem Sterilisationsbehälter zu sorgen, ist es vorteilhaft, wenn der Innendurchmesser des Ventilsitzes des Dampfeintrittsventils im Wesentlichen dem Außendurchmesser des rohrförmigen Dampfauslasses entspricht. Demnach wird der rohrförmige Dampfauslass im Wesentlichen entlang der Innenseite des Ventilsitzes des Dampfeintrittsventils verschoben, während der Sterilisationsbehälter mit der Abdeckplatte verbunden wird.

Um ein manuelles Öffnen bzw. Schließen des Dampfeintrittsventils zu ermöglichen, ist es vorteilhaft, wenn der Ventilkörper des Dampfeintrittsventils an der Oberseite ein Griffelement, insbesondere eine Grifflasche, zur manuellen Verschiebung des Ventilkörpers zwischen der Offen- und der Schließstellung aufweist. Diese Ausführung ist besonders vorteilhaft, wenn der Sterilisationsbehälter allein, ohne die Wasseraufnahmeeinheit mit der Abdeckplatte und die Dampferzeugungseinheit, verwendet werden soll. Für solche Anwendungen kann der Benutzer das Dampfeintrittsventil manuell zwischen der Offen- und der Schließstellung verschieben, so dass ein ungewollter Dampfaustritt, insbesondere bei einer Erhitzung in einer Mikrowelle, über das Dampfeintrittsventil verhindert wird. Auch für eine Kaltsterilisation ist diese Ausführung vorteilhaft. Andererseits kann das Dampfeintrittsventil auch so ausgestaltet sein, dass der verschiebliche Ventilkörper bei der Abnahme des Sterilisationsbehälters vom rohrförmigen Dampfauslass der Abdeckplatte durch das Eigengewicht des Ventilkörpers selbsttätig in die Schließstellung verschoben wird.

Um den rohrförmigen Dampfauslass und das Dampfeintrittsventil in einer definierten Stellung zueinander anzuordnen, ist es vorteilhaft, wenn der rohrförmige Dampfauslass der Abdeckplatte an der Oberseite einen Justierflansch aufweist, wobei in der zweiten Funktionsstellung der Ventilkörper des Dampfeintrittsventils zwischen dem Justierflansch des rohrförmigen Dampfauslasses und dem Ventilsitz des Dampfeintrittsventils angeordnet ist. Vorzugsweise ist ein ringförmiger Justierflansch vorgesehen, welcher an der Innenseite eines hohlen Ventilkörpers anliegt.

Zum Abnehmen der Abdeckplatte von der Wasseraufnahmeeinheit ist es günstig, wenn die Abdeckplatte insbesondere einteilig mit einem Griffteil verbunden ist.

Bei einer Verwendung der erfindungsgemäßen Vorrichtung in der ersten Funktionsstellung ist es von Vorteil, wenn die Trageinheit zumindest eine Durchtrittsöffnung zum Beaufschlagen des Babyartikels mit Wasserdampf aus der Wasseraufnahmeeinheit aufweist. In der ersten Funktionsstellung ist die Trageinheit als modulares Aufsatzelement auf der Wasseraufnahmeeinheit angeordnet, wobei der Wasserdampf aus der Wasseraufnahmeeinheit über die Durchtrittsöffnung zu dem Babyartikel gelangt. Bevorzugt weist die Trageinheit mehrere, insbesondere großflächige Durchtrittsöffnungen für den Wasserdampf auf.

Um die erfindungsgemäße Vorrichtung alternativ mit der Abdeckplatte und dem Sterilisationsbehälter einerseits oder mit der Trageinheit andererseits verwenden zu können, ist es von Vorteil, wenn ein die Wasseraufnahmeeinheit aufnehmendes Gehäuse vorgesehen ist, welches einen Halteabschnitt zur im Wesentlichen dampfdichten Verbindung wahlweise mit der Trageinheit oder der Abdeckplatte aufweist. Im verbundenen Zustand können daher keine bzw. allenfalls geringe Mengen an Wasserdampf aus dem Wasseraufnahmebehälter über den Halteabschnitt des Gehäuses nach außen entweichen, so dass vorteilhafterweise im Wesentlichen der gesamte Wasserdampf entweder der Trageinheit oder dem rohrförmigen Dampfauslass der Abdeckplatte zuführbar ist.

Gemäß einer besonders bevorzugten Ausführung ist vorgesehen, dass die Trageinheit und/oder die Abdeckplatte jeweils ein Verbindungselement zur Steck-Dreh-Verbindung mit einer Aufnahmeöffnung am Halteabschnitt des Gehäuses aufweist. Demnach ist die Trageinheit in der ersten Funktionsstellung bzw. die Abdeckplatte in der zweiten Funktionsstellung in der Art einer Bajonettverbindung mit dem Halteabschnitt am Gehäuse verbunden. Zu diesem Zweck weist der Halteabschnitt eine längliche Aufnahmeöffnung auf, in welcher das Verbindungselement der Trageinheit bzw. der Abdeckplatte durch eine Steck-Dreh-Bewegung lösbar aufnehmbar ist. Die Aufnahmeöffnung weist einen zur Trageinheit bzw. Abdeckplatte hin offenen Einlaufabschnitt und einen dazu abgewinkelten, bevorzugt schlitzförmigen Sicherungsabschnitt auf, welcher am Ende durch einen Anschlag begrenzt ist. Bevorzugt weist der Halteabschnitt des Gehäuses zumindest zwei, insbesondere gegenüberliegend am Gehäuse angeordnete, Aufnahmeöffnungen auf, deren Sicherungsabschnitte in entgegengesetzte Richtungen erstreckt sind. Dadurch wird eine schnell und einfache herzustellende bzw. zu lösende Verbindung geschaffen, welche zudem nicht zu Verschleiß neigt.

Darüber hinaus ist es günstig, wenn die Trageinheit und/oder die Abdeckplatte jeweils ein Aufsetzelement zum Aufsetzen auf den Halteabschnitt des Gehäuses aufweist, wobei das Aufsetzelement vorzugsweise einen im Wesentlichen horizontalen Auflageflansch zum Aufsetzen auf eine entsprechende Auflagefläche des Halteabschnitts des Gehäuses aufweist. Durch die Aufsetzelemente können die Trageinheit und die Abdeckplatte als Aufsetzmodule in einer definierten Stellung auf dem Gehäuse für die Wasseraufnahmeeinheit aufgesetzt werden. Dadurch kann die Trageinheit in der ersten Funktionsstellung bzw. die Abdeckplatte in der zweiten Funktionsstellung auf stabile Weise mit dem Gehäuse verbunden werden. Bevorzugt sind sowohl die zuvor beschriebenen Steck-Dreh-Verbindungselemente in der Art einer Bajonettverbindung als auch die Aufsetzelemente vorgesehen.

Um einen unerwünschten Dampfaustritt aus der Vorrichtung zuverlässig zu verhindern, ist bevorzugt vorgesehen, dass in der ersten und/oder zweiten Funktionsstellung ein Dichtelement zwischen der Trageinheit bzw. der Abdeckplatte und dem Halteabschnitt des Gehäuses angeordnet ist. Bevorzugt ist das Dichtelement an dem Halteabschnitt des Gehäuses vorgesehen. Im verbundenen Zustand liegt die Abdeckplatte an dem Dichtelement des Gehäuses an, so dass ein Dampfaustritt in die Umgebung im Wesentlichen vollständig unterbunden wird. In der zweiten Funktionsstellung kann daher im Wesentlichen der gesamte Dampf aus der Wasseraufnahmeeinheit durch das Dampfeintrittsventil in den Sterilisationsbehälter geleitet werden.

Um die gewünschte Dichtwirkung zu erzielen, ist es günstig, wenn als Dichtelement ein insbesondere im Wesentlichen kreisförmig umlaufender Dichtflansch am Halteabschnitt des Gehäuses vorgesehen ist, an welchem in der ersten und/oder zweiten Funktionsstellung ein entsprechender, insbesondere im Wesentlichen kreisförmig umlaufender, Anlageflansch der Trageinheit bzw. der Abdeckplatte anliegt. Besonders bevorzugt ist hierbei, wenn die Trageinheit bzw. die Abdeckplatte über die zuvor beschriebene Steck-Dreh-Verbindung mit dem Halteabschnitt des Gehäuses verbindbar ist, wobei im verbundenen Zustand der Anlageflansch der Trageinheit bzw. der Abdeckplatte in dichtenden Kontakt mit dem Dichtflansch des Gehäuses tritt.

Die der Erfindung zugrundeliegende Aufgabe wird zudem durch einen Sterilisationsbehälter der eingangs angeführten Art gelöst, bei welchem das Bodenteil ein Dampfeintrittsventil mit einer Dampfeintrittsöffnung aufweist, wobei das Dampfeintrittsventil von einer die Dampfeintrittsöffnung verschließenden Schließstellung mittels des rohrförmigen Dampfauslasses der Abdeckplatte der Wasseraufnahmeeinheit (3) in eine die Dampfeintrittsöffnung freigebenden Offenstellung überführbar ist.

Die Vorteile und technischen Effekte dieser Ausführung gehen bereits aus den vorstehenden Erläuterungen hervor. Demnach kann der Sterilisationsbehälter einerseits mit einer an eine Dampferzeugungseinheit angeschlossenen Wasseraufnahmeeinheit verwendet werden, wobei das Dampfeintrittsventil in der Offenstellung angeordnet ist. Andererseits kann der Sterilisationsbehälter bei geschlossenem Dampfeintrittsventil mit einer bestimmten Wassermenge gefüllt und in einer Mikrowelle platziert werden. Im Betrieb der Mikrowelle wird das Wasser verdampft, wobei der Babyartikel in dem Aufnahmeraum mit dem entstehenden Wasserdampf beaufschlagt wird.

Wie bereits zuvor beschrieben, ist es hierbei günstig, wenn das Dampfeintrittsventil einen an einem stutzenförmigen Ventilsitz verschieblich gelagerten Ventilkörper aufweist. Das Dampfeintrittsventil ist dazu eingerichtet, dass es beim Verbinden mit einem rohrförmigen Dampfauslass selbsttätig in die Offenstellung gebracht wird.

Vorzugsweise ist eine Abdeckplatte vorgesehen, bei welcher ein rohrförmiger Dampfauslass zur Verbindung mit einem Dampfeintrittsventil eines Sterilisationsbehälters vorgesehen ist.

Die Abdeckplatte und deren Vorteile bei einer Verwendung in der erfindungsgemäßen Vorrichtung wurden bereits oben in Verbindung mit der erfindungsgemäßen Vorrichtung dargelegt. Bevorzugt weist die Abdeckplatte eine scheibenförmige Grundform auf, an welcher der rohrförmige Dampfauslass ausgebildet ist. Gegebenenfalls kann die Abdeckplatte zudem ein Aufsetzelement zum Aufsetzen auf ein Gehäuse für eine Wasseraufnahmeeinheit aufweisen. Das Aufsetzelement weist bevorzugt einen horizontalen, d.h. in der Hauptebene der Abdeckplatte erstreckten, Auflageflansch und einen vertikalen, d.h. senkrecht zur Hauptebene der Abdeckplatte erstreckten, Anlageflansch aufweist.

Die Erfindung wird nachstehend anhand eines in der Zeichnung dargestellten bevorzugten Ausführungsbeispiels, auf das sie jedoch nicht beschränkt sein soll, noch weiter erläutert. Im Einzelnen zeigen in der Zeichnung:
Fig. 1 eine Schnittansicht einer erfindungsgemäßen Vorrichtung zum Erwärmen bzw. Sterilisieren eines Babyartikels mit einer Dampferzeugungseinheit und mit einer Wasseraufnahmeeinheit, wobei die Wasseraufnahmeeinheit in der gezeigten ersten Funktionsstellung mit einer Trageinheit mit einer Aufstandsfläche für den Babyartikel verbunden ist;
Fig. 2a eine schaubildliche, teilweise geschnittene Ansicht der erfindungsgemäßen Vorrichtung gemäß Fig. 1, wobei die Wasseraufnahmeeinheit in der gezeigten zweiten Funktionsstellung anstelle der Trageinheit mit einer Abdeckplatte mit einem rohrförmigen Dampfauslass verbunden ist, auf welche ein Sterilisationsbehälter mit einem Dampfeintrittsventil aufgesetzt ist;
Fig. 2b eine schaubildliche Ansicht eines die Wasseraufnahmeeinheit aufnehmenden Gehäuses, welches eine Aufnahmeöffnung zur Steck-Dreh-Verbindung mit einem Verbindungselement der Abdeckplatte aufweist;
Fig. 2c eine Detailansicht der Verbindung zwischen der Abdeckplatte und dem Gehäuse in der zweiten Funktionsstellung gemäß Fig. 2a, 2b;
Fig. 3 eine Detailansicht der Vorrichtung in der zweiten Funktionsstellung gemäß Fig. 2a im Bereich des Dampfeintrittsventils des Sterilisationsbehälters; und
Fig. 4 eine Schnittansicht des Sterilisationsbehälters gemäß Fig. 2a in einer Schließstellung des Dampfeintrittsventils, um eine Sterilisation des Babyartikels, beispielsweise in einer Mikrowelle, vorzunehmen.

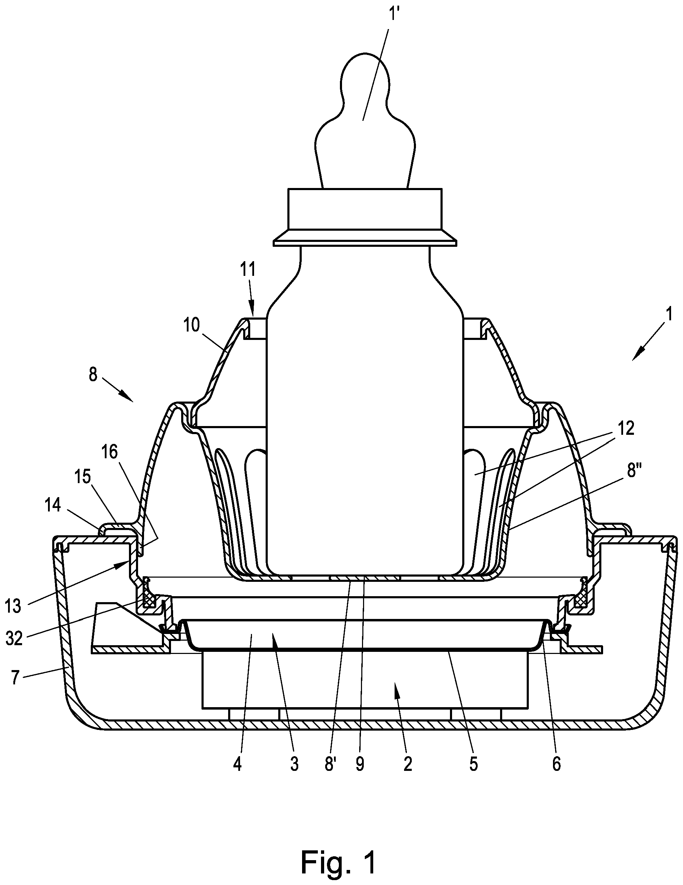

Die Zeichnungen zeigen eine Vorrichtung 1 zum Erwärmen bzw. Sterilisieren eines Babyartikels, wobei in Fig. 1 schematisch eine Babyflasche 1' ersichtlich ist. Die Vorrichtung 1 weist eine elektrische Dampferzeugungseinheit 2 auf, welche in herkömmlicher Art und Weise ausgebildet sein kann, so dass auf nähere Erläuterungen hierzu verzichtet werden kann. Darüber hinaus ist eine Wasseraufnahmeeinheit 3 vorgesehen, welche einen wannen- bzw. beckenförmigen Wasseraufnahmebehälter 4 zum Befüllen mit Wasser aufweist. Der Wasseraufnahmebehälter 4 weist einen Boden 5 und eine davon hochstehende Seitenwand 6 auf, welche ein nach oben offenes Wasserbecken bilden. Die Wasseraufnahmeeinheit 4 ist mit der Dampferzeugungseinheit 2 derart verbunden, dass im Betrieb eine in dem Wasseraufnahmebehälter 4 vorgesehene Wassermenge verdampft werden kann. Weiters weist die Vorrichtung 1 ein schalenförmiges Gehäuse 7 auf, in welchem die Dampferzeugungseinheit 2 und die Wasseraufnahmeeinheit 4 angeordnet sind. In dem Gehäuse 7 ist eine Elektrik zum Betrieb der Dampferzeugungseinheit 2 aufgenommen (nicht gezeigt).

Gemäß Fig. 1 ist die Wasseraufnahmeeinheit 4 in einer ersten Funktionsstellung modulartig mit einer Trageinheit 8 verbunden, welche haubenförmig auf das Gehäuse 7 der Vorrichtung 1 aufgesetzt ist. Die Trageinheit 8 weist einen Bodenabschnitt 8' und einen umlaufenden Seitenwandabschnitt 8'' auf, welche eine Aufnahmekammer für die Babyflasche 1' einschließen. Am Bodenabschnitt 8' ist eine Aufstandsfläche 9 zum Aufstellen der Babyflasche 1' ausgebildet. An der Oberseite der Trageinheit 8 ist ein abnehmbares Deckelelement 10 vorgesehen, welches eine zentrale Öffnung 11 zum Einstellen des Babyartikels aufweist. In der gezeigten Ausführung ist die Babyflasche 1' mit einem geringen Freiraum bzw. Spiel innerhalb der zentralen Öffnung 11 des Deckelelements 10 angeordnet, wodurch eine vorteilhafte Dampfleitung zum Erwärmen des Inhalts der Babyflasche 1' erzielt wird.

Wie aus Fig. 1 weiters ersichtlich, weist die Trageinheit 8 mehrere längliche Durchtrittsöffnungen 12 zum Beaufschlagen des Babyartikels mit Wasserdampf aus dem Wasseraufnahmebehälter 4 auf. Die Durchtrittsöffnungen 12 erstrecken sich von dem umlaufenden Seitenwandabschnitt 8'' bis in den Bodenabschnitt 8'. In Gebrauch wird Dampf aus dem Wasseraufnahmebehälter 4 über die Durchtrittsöffnungen 12 in die Aufnahmekammer der Trageinheit 8 gefördert, um die Babyflasche 1' mit dem heißen Dampf zu beaufschlagen und dadurch zu erwärmen.

Wie aus Fig. 1 weiters ersichtlich, weist das die Wasseraufnahmeeinheit 3 aufnehmende Gehäuse 7 an der Oberseite einen umlaufenden Halteabschnitt 13 zur Anordnung der Trageinheit 8 auf. Zu diesem Zweck weist die Trageinheit 8 ein Aufsetzelement 14 auf, mit welchem die Trageinheit 8 auf dem Halteabschnitt 13 des Gehäuses 7 aufsetzbar ist. Das Aufsetzelement 14 weist einen im Wesentlichen horizontalen Auflageflansch 15 zum Aufsetzen auf eine entsprechende Auflagefläche des Halteabschnitts 13 des Gehäuses 7 und einen im Wesentlichen vertikalen Anlageflansch 16 zur Anlage an einer entsprechenden Anlagefläche des Halteabschnitts 13 des Gehäuses 7 auf.

Für die Zwecke dieser Offenbarung beziehen sich die Begriffe "vertikal", "horizontal", "Oberseite" und "Unterseite" auf eine Betriebsstellung der Vorrichtung 1, bei welcher die Vorrichtung 1 auf einer horizontalen Abstellfläche angeordnet ist.

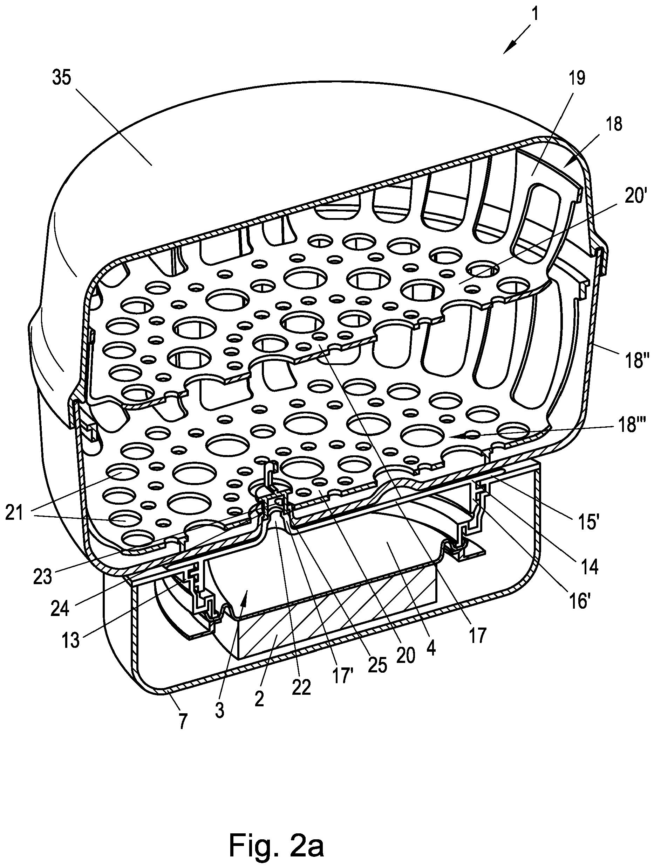

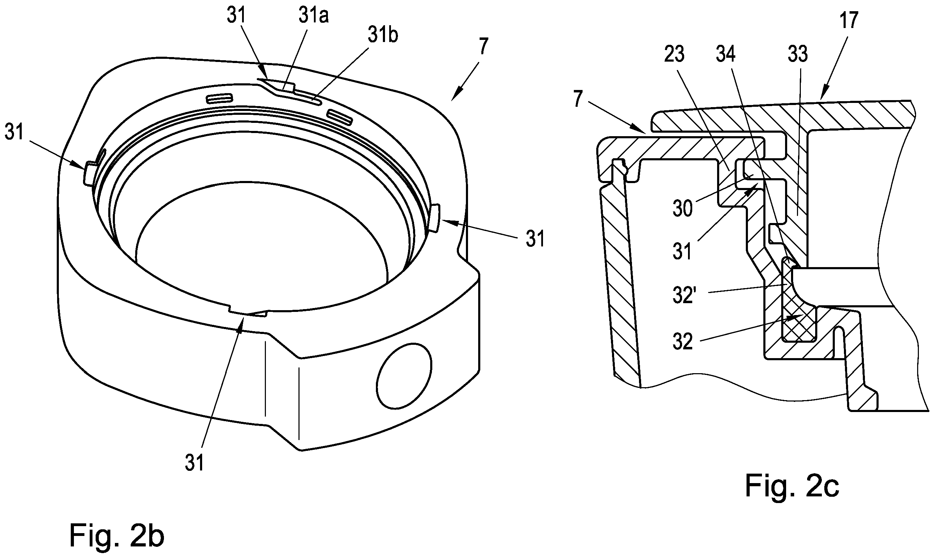

Wie aus Fig. 2a ersichtlich, kann der Halteabschnitt 13 des Gehäuses 7 in einer zweiten Funktionsstellung alternativ zur Trageinheit 8 mit einer Abdeckplatte 17 verbunden werden. In der gezeigten Ausführung weist die Abdeckplatte 17 ein zapfen- bzw. flanschförmiges Verbindungselement 30 (vgl. Fig. 2c) zur Steck-Dreh-Verbindung mit einer Aufnahmeöffnung 31 am Halteabschnitt 23 des Gehäuses 7 auf. Demnach ist zwischen der Abdeckplatte 17 und dem Halteabschnitt 23 am Gehäuse 7 eine werkzeuglos lösbare Bajonettverbindung vorgesehen.

Wie aus Fig. 2b ersichtlich, weist die Aufnahmeöffnung 31 einen nach oben, zur Abdeckplatte 17 hin offenen Einlaufabschnitt 31a und einen im Wesentlichen horizontal verlaufenden, schlitzförmigen Sicherungsabschnitt 31b auf, welcher durch einen Anschlag begrenzt ist. Demnach ist die Abdeckplatte 17 über das Verbindungselement 30 durch eine Steckbewegung entlang des Einlaufabschnitts 31 und eine Drehbewegung entlang des Sicherungsabschnitts 31b am Halteabschnitt 23 festlegbar. In der gezeigten Ausführung weist der Halteabschnitt 23 des Gehäuses 7 mehrere, gegenüberliegend am Gehäuse 7 angeordnete Aufnahmeöffnungen 31 auf, deren Sicherungsabschnitte 31b paarweise in entgegengesetzte Richtungen erstreckt sind.

Wie aus Fig. 2a ersichtlich, vgl. insbesondere auch die Detailansicht der Fig. 2c, weist der Halteabschnitt 23 am Gehäuse 7 ein Dichtelement 32 zur Abdichtung gegenüber der Abdeckplatte 17 auf. Als Dichtelement 32 ist in der gezeigten Ausführung ein im Wesentlichen kreisförmiger Dichtflansch 32' vorgesehen, an welchem in der zweiten Funktionsstellung gemäß Fig. 2a ein entsprechender, ebenfalls kreisförmiger Anlageflansch 33 der Abdeckplatte 17 dichtend anliegt. Zu diesem Zweck weist der Dichtflansch am oberen Ende eine Dichtlippe 34 auf, welche beim Herstellen der Steck-Dreh-Verbindung in dichtenden Kontakt mit dem unteren Ende des Anlageflansches 33 tritt. Dadurch ist eine im Wesentlichen dampfdichte Verbindung zwischen der Abdeckplatte 17 und dem Halteabschnitt 13 des Gehäuses 7 vorgesehen, so dass ein Dampfaustritt in die Umgebung im Wesentlichen vollständig gesperrt wird.

Die Trageinheit 8 gemäß Fig. 1 kann in derselben Weise wie die Abdeckplatte 17 über eine Steck-Dreh-Verbindung mit dem Gehäuse 7 verbunden sein (nicht gezeigt).

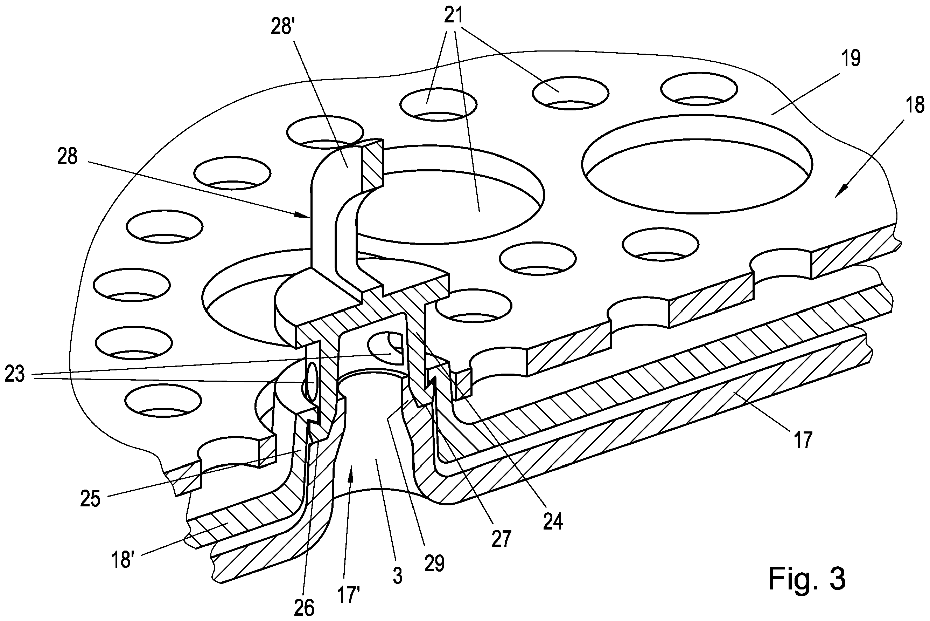

Wie aus Fig. 2a ersichtlich, weist die Abdeckplatte 17 einen rohrförmigen Dampfauslass 17' auf, mit welchem der Wasserdampf aus der zwischen dem Wasseraufnahmebehälter 4 und der Abdeckplatte 17 gebildeten Dampfkammer abtransportiert wird. Die Abdeckplatte 17 ist in der zweiten Funktionsstellung mit einem Sterilisationsbehälter 18 verbunden. Der Sterilisationsbehälter 18 weist einen Bodenteil 18' (vgl. auch Fig. 3), einen umlaufenden Seitenteil 18'' und einen Deckelteil 35 auf, welche einen Aufnahmeraum 18''' für die Babyflasche 1' einschließen. In dem Sterilisationsbehälter 18 ist ein Einsatzelement 19 herausnehmbar angeordnet. Der Einsatzteil 19 weist eine untere Auflagefläche 20 und eine obere Auflagefläche 20' zur Auflage von Babyartikeln 1 auf. In dem Einsatzteil 19 sind zudem verschiedenartige Durchgangsöffnungen 21 ausgebildet, welche einen Durchtritt von Wasser bzw. Wasserdampf ermöglichen. In der zweiten Funktionsstellung gemäß Fig. 2 ist der rohrförmige Dampfauslass 17' der Abdeckplatte 17 mit einem Dampfeintrittsventil 22 des Sterilisationsbehälters 18 verbunden, welches in Fig. 3 in vergrößertem Maßstab ersichtlich ist.

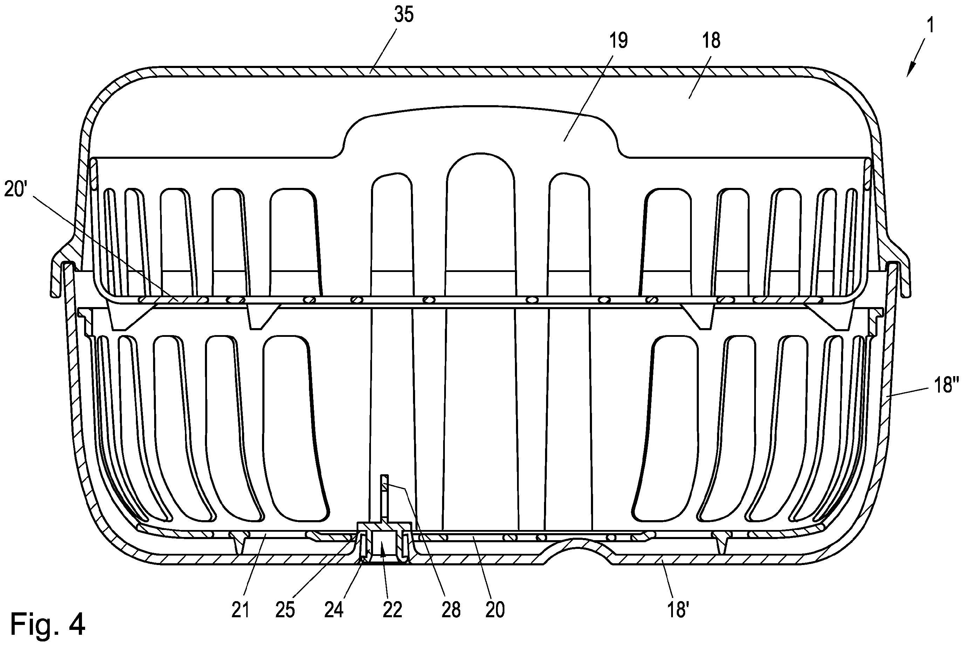

Wie aus Fig. 2, 3 ersichtlich, ist das Dampfeintrittsventil 22 des Sterilisationsbehälters 18 mittels des rohrförmigen Dampfauslasses 17' der Abdeckplatte 17 aus einer mehrere Dampfeintrittsöffnungen 23 verschließenden Schließstellung (vgl. Fig. 4) in eine die Dampfeintrittsöffnungen 23 freigebenden Offenstellung (vgl. Fig. 2, 3) überführbar. Hiefür weist das Dampfeintrittsventil 22 des Sterilisationsbehälters 18 einen hülsenförmigen Ventilkörper 24 auf, welcher an einem stutzenförmigen Ventilsitz 25 verschieblich gelagert ist.

Wie aus Fig. 2, 3 weiters ersichtlich, weist der Ventilkörper 24 des Dampfeintrittsventils 22 an der der Abdeckplatte 17 zugewandten Unterseite eine ringförmig umlaufende Anschlagfläche 26 auf, welcher eine entsprechende Anschlagfläche 27 an der von der Wasseraufnahmeeinheit 3 abgewandten Oberseite des rohrförmigen Dampfauslasses 17' zugeordnet ist. Der stutzenförmige Ventilsitz 25 des Dampfeintrittsventils 22 springt im Wesentlichen senkrecht von dem Bodenteil 18' des Sterilisationsbehälters 18 in den Aufnahmeraum 18''' für die Babyflasche 1' vor. Entsprechend steht der rohrförmige Dampfauslass 17' im Wesentlichen senkrecht von der Hauptebene der Abdeckplatte 17 hoch. Dadurch wird der Ventilköper 24 des Dampfeintrittsventils 22 durch die Anordnung des rohrförmigen Dampfauslasses 17' der Abdeckplatte 17 selbsttätig in die in Fig. 2, 3 gezeigte Offenstellung gebracht, in welcher die an der Mantelfläche des Ventilkörpers 24 vorgesehenen Dampfeintrittsöffnungen 23 oberhalb der Oberkante des stutzenförmigen Ventilsitzes 25 angeordnet sind und daher einen Dampfdurchtritt ermöglichen.

Wie aus Fig. 2, 3 weiters ersichtlich, entspricht der Innendurchmesser des Ventilsitzes 25 des Dampfeintrittsventils 22 im Wesentlichen dem Außendurchmesser des rohrförmigen Dampfauslasses 17'. Für eine manuelle Bedienung weist der Ventilkörper 24 des Dampfeintrittsventils 22 an der Oberseite ein Griffelement 28 auf, welches in der gezeigten Ausführung durch eine Grifflasche 28' gebildet ist. Die Abdeckplatte 17 kann ebenfalls ein (nicht ersichtliches) Griffteil zum Abnehmen der Abdeckplatte 17 von der Wasseraufnahmeeinheit 3 aufweisen.

Weiters ist aus Fig. 2, 3 ersichtlich, dass der rohrförmige Dampfauslass 17' der Abdeckplatte 17 an der Oberseite einen Justierflansch 29 aufweist. In der in Fig. 2, 3 ersichtlichen zweiten Funktionsstellung ist der Ventilkörper 24 des Dampfeintrittsventils 22 zwischen dem Justierflansch 29 des rohrförmigen Dampfauslasses 17' und dem Ventilsitz 25 des Dampfeintrittsventils 22 angeordnet.

Wie aus einem Vergleich zwischen der ersten Funktionsstellung gemäß Fig. 1 und der zweiten Funktionsstellung gemäß Fig. 2, 3 ersichtlich, weist der Sterilisationsbehälter 18 eine geringere Höhe als die Trageinheit 8 auf. Dies hängt mit dem unterschiedlichen Verwendungszweck zusammen, da die Trageinheit 8 bevorzugt zum Erwärmen des Inhalts einer stehenden Babyflasche und der Sterilisationsbehälter 18 zum Sterilisieren bzw. Desinfizieren einer liegenden Babyflasche verwendet wird.

Üblicherweise werden derartige Vorrichtungen Sterilisationsvorrichtungen ("sterilizer") genannt, wobei auch diese Vorrichtungen tatsächlich nur eine Desinfektion (=Keimzahlreduktion um den Fakter 10⁵) und nicht eine Sterilisation (=Keimzahlreduktion um den Fakter 10⁶) durchführen. Nachfolgend werden die beiden Begriffe jedoch synonym verwendet, und es ist hiermit eine Keimzahlreduktion zumindest um den Faktor 10⁵ gemeint.

Fig. 4 zeigt eine weitere Verwendungsmöglichkeit des Sterilisationsbehälters 18, welcher bei geschlossenem Dampfeintrittsventil 22 zur Sterilisation bzw. Desinfektion von Babyartikeln mittels einer Mikrowelle genutzt werden kann. Durch den stutzenförmigen Ventilsitz 25 ist sichergestellt, dass eine ausreichende Wassermenge in den Sterilisationsbehälter 18 gefüllt werden kann, welche nicht über das Dampfeintrittsventil 22 auslaufen kann. Andererseits wird ein Austritt des bei der Erhitzung in der Mikrowelle entstehenden Wasserdampfes in der Schließstellung des Dampfeintrittsventils 22 weitgehend unterbunden.

## Patentansprüche

1. Vorrichtung (1) zum Erwärmen bzw. Sterilisieren eines Babyartikels (1') mit einer Dampferzeugungseinheit (2), welche mit einer wannenförmigen Wasseraufnahmeeinheit (3) verbunden ist, wobei die Wasseraufnahmeeinheit (3) in einer ersten Funktionsstellung mit einer Trageinheit (8) mit einer Aufstandsfläche (9) für den Babyartikel verbunden ist, **dadurch gekennzeichnet** dassin einer zweiten Funktionsstellung alternativ mit einer Abdeckplatte (17) mit einem rohrförmigen Dampfauslass (17') verbunden ist, wobei die Abdeckplatte (17) in der zweiten Funktionsstellung mit einem Sterilisationsbehälter (18) zur Aufnahme des Babyartikels verbunden ist und der rohrförmige Dampfauslass (17') der Abdeckplatte (17) mit einem Dampfeintrittsventil (22) des Sterilisationsbehälters (18) in Verbindung steht.

2. Vorrichtung (1) nach Anspruch 1, **dadurch gekennzeichnet, dass** das Dampfeintrittsventil (22) des Sterilisationsbehälters (18) mittels des rohrförmigen Dampfauslasses (17') der Abdeckplatte (17) aus einer eine Dampfeintrittsöffnung (23) verschließenden Schließstellung in eine die Dampfeintrittsöffnung (23) freigebenden Offenstellung überführbar ist.

3. Vorrichtung (1) nach Anspruch 2, **dadurch gekennzeichnet, dass** das Dampfeintrittsventil (22) des Sterilisationsbehälters (18) einen an einem stutzenförmigen Ventilsitz (25) verschieblich gelagerten Ventilkörper (24) aufweist, wobei der Ventilköper (24) durch die Anordnung des rohrförmigen Dampfauslasses (17') der Abdeckplatte (17) in der Offenstellung angeordnet ist.

4. Vorrichtung (1) nach Anspruch 3, **dadurch gekennzeichnet, dass** der Ventilkörper (24) des Dampfeintrittsventils (22) an der Unterseite eine umlaufende Anschlagfläche (26) aufweist, welche mit einer entsprechenden Anschlagfläche (27) an der Oberseite des rohrförmigen Dampfauslasses (17') zusammenwirkt.

5. Vorrichtung (1) nach Anspruch 3 oder 4, **dadurch gekennzeichnet, dass** der stutzenförmige Ventilsitz (25) des Dampfeintrittsventils (22) im Wesentlichen senkrecht von einem Bodenteil (18') des Sterilisationsbehälters (18) nach innen vorspringt, wobei der rohrförmige Dampfauslass (17') im Wesentlichen senkrecht von der Hauptebene der Abdeckplatte (17) hochsteht.

6. Vorrichtung (1) nach einem der Ansprüche 3 bis 5, **dadurch gekennzeichnet, dass** der Innendurchmesser des Ventilsitzes (25) des Dampfeintrittsventils (22) im Wesentlichen dem Außendurchmesser des rohrförmigen Dampfauslasses (17') entspricht.

7. Vorrichtung (1) nach einem der Ansprüche 3 bis 6, **dadurch gekennzeichnet, dass** der Ventilkörper (24) des Dampfeintrittsventils (17) an der Oberseite ein Griffelement (28) zur manuellen Verschiebung des Ventilkörpers (24) zwischen der Offen- und der Schließstellung aufweist.

8. Vorrichtung (1) nach einem der Ansprüche 3 bis 7, **dadurch gekennzeichnet, dass** der rohrförmige Dampfauslass (17') der Abdeckplatte (17) an der Oberseite einen Justierflansch (29) aufweist, wobei in der zweiten Funktionsstellung der Ventilkörper (24) des Dampfeintrittsventils (22) zwischen dem Justierflansch (29) des rohrförmigen Dampfauslasses (17') und dem Ventilsitz (25) des Dampfeintrittsventils (22) angeordnet ist.

9. Vorrichtung (1) nach Anspruch 1, **dadurch gekennzeichnet, dass** die Abdeckplatte (17) mit einem Griffteil zum Abnehmen der Abdeckplatte (17) von der Wasseraufnahmeeinheit (3) verbunden ist.

10. Vorrichtung (1) nach einem der Ansprüche 1 bis 9, **dadurch gekennzeichnet, dass** die Trageinheit (8) zumindest eine Durchtrittsöffnung (12) zum Beaufschlagen des Babyartikels mit Wasserdampf aus der Wasseraufnahmeeinheit (3) aufweist.

11. Vorrichtung (1) nach einem der Ansprüche 1 bis 10, **dadurch gekennzeichnet, dass** ein die Wasseraufnahmeeinheit (3) aufnehmendes Gehäuse (7) vorgesehen ist, welches einen Halteabschnitt (23) zur im Wesentlichen dampfdichten Verbindung wahlweise mit der Trageinheit (8) oder der Abdeckplatte (17) aufweist.

12. Vorrichtung (1) nach Anspruch 11, **dadurch gekennzeichnet, dass** die Trageinheit (8) und/oder die Abdeckplatte (17) jeweils ein Verbindungselement (30) zur Steck-Dreh-Verbindung mit einer Aufnahmeöffnung am Halteabschnitt (23) des Gehäuses (7) aufweist.

13. Vorrichtung (1) nach Anspruch 11 oder 12, **dadurch gekennzeichnet, dass** die Trageinheit (8) und/oder die Abdeckplatte (17) jeweils ein Aufsetzelement (14) zum Aufsetzen auf den Halteabschnitt (23) des Gehäuses (7) aufweist, wobei das Aufsetzelement (14) einen horizontalen Auflageflansch (15, 15') zum Aufsetzen auf eine entsprechende Auflagefläche des Halteabschnitts (23) des Gehäuses (7) der Wasseraufnahmeeinheit (3) aufweist.

14. Vorrichtung (1) nach einem der Ansprüche 11 bis 13, **dadurch gekennzeichnet, dass** in der ersten und/oder zweiten Funktionsstellung ein Dichtelement zwischen der Trageinheit (8) bzw. der Abdeckplatte (17) und dem Halteabschnitt (23) des Gehäuses (7) angeordnet ist.

15. Vorrichtung (1) nach Anspruch 14, **dadurch gekennzeichnet, dass** als Dichtelement ein Dichtflansch am Halteabschnitt (23) des Gehäuses (7) vorgesehen ist, an welchem in der ersten und/oder zweiten Funktionsstellung ein entsprechender Anlageflansch (16, 16') der Trageinheit (8) bzw. der Abdeckplatte (17) anliegt.

## Claims

1. Device (1) for heating or sterilising a baby item (1'), comprising a steam-generating unit (2) connected to a basin-shaped water receiving unit (3), the water receiving unit (3), in a first operating position, being connected to a carrier unit (8) that has a first contact surface (9) for the baby item, **characterised in that**, in a second operating position, the water receiving unit is alternatively connected to a cover plate (17) that has a tubular steam outlet (17'), the cover plate (17) being connected, in the second operating position, to a sterilisation container (18) for receiving the baby item, and the tubular steam outlet (17') of the cover plate (17) being connected to a steam inlet valve (22) of the sterilisation container (18).

2. Device (1) according to claim 1, **characterised in that** the steam inlet valve (22) of the sterilisation container (18) can be moved, by means of the tubular steam outlet (17') of the cover plate (17), out of a closed position that closes the steam inlet opening (23) and into an open position that releases the steam inlet opening (23).

3. Device (1) according to claim 2, **characterised in that** the steam inlet valve (22) of the sterilisation container (18) comprises a valve body (24) that is movably mounted on a nozzle-shaped valve seat (25), the valve body (24) being arranged in the open position by means of the arrangement of the tubular steam outlet (17') of the cover plate (17).

4. Device (1) according to claim 3, **characterised in that** the valve body (24) of the steam inlet valve (22) comprises a peripheral stop face (26) on the underside thereof, which face interacts with a corresponding stop face (27) on the top of the tubular steam outlet (17').

5. Device (1) according to either claim 3 or claim 4, **characterised in that** the nozzle-shaped valve seat (25) of the steam inlet valve (22) projects substantially perpendicularly inwards from a base part (18') of the sterilisation container (18), the tubular steam outlet (17') extending upwards substantially perpendicularly from the main plane of the cover plate (17).

6. Device (1) according to any of claims 3 to 5, **characterised in that** the inside diameter of the valve seat (25) of the steam inlet valve (22) substantially corresponds to the outside diameter of the tubular steam outlet (17').

7. Device (1) according to any of claims 3 to 6, **characterised in that** the valve body (24) of the steam inlet valve (17) comprises a handle element (28) on the top thereof for manually moving the valve body (24) between the open position and the closed position.

8. Device (1) according to any of claims 3 to 7, **characterised in that** the tubular steam outlet (17') of the cover plate (17) comprises an adjustment flange (29) on the top thereof, the valve body (24) of the steam inlet valve (22) being arranged between the adjustment flange (29) of the tubular steam outlet (17') and the valve seat (25) of the steam inlet valve (22) when in the second operating position.

9. Device (1) according to claim 1, **characterised in that** the cover plate (17) is connected to a handle part for removing the cover plate (17) from the water receiving unit (3).

10. Device (1) according to any of claims 1 to 9, **characterised in that** the carrier unit (8) comprises at least one passage (12) for applying steam from the water receiving unit (3) to the baby item.

11. Device (1) according to any of claims 1 to 10, **characterised in that** a housing (7) that receives the water receiving unit (3) is provided, which housing comprises a retaining portion (23) for substantially steam-tight connection to the carrier unit (8) or the cover plate (17), as desired.

12. Device (1) according to claim 11, **characterised in that** the carrier unit (8) and/or the cover plate (17) each comprise a connection element (30) for a plug and twist connection to a receiving opening on the retaining portion (23) of the housing (7).

13. Device (1) according to either claim 11 or claim 12, **characterised in that** the carrier unit (8) and/or the cover plate (17) each comprise a placement element (14) for being placed on the retaining portion (23) of the housing (7), the placement element (14) comprising a horizontal support flange (15, 15') for being placed on a corresponding support surface of the retaining portion (23) of the housing (7) of the water receiving unit (3).

14. Device (1) according to any of claims 11 to 13, **characterised in that** in the first and/or the second operating position, a sealing element is arranged between the carrier unit (8) or the cover plate (17) and the retaining portion (23) of the housing (7).

15. Device (1) according to claim 14, **characterised in that** a sealing flange is provided on the retaining portion (23) of the housing (7) as a sealing element, on which flange a corresponding contact flange (16, 16') of the carrier unit (8) or of the cover plate (17) rests in the first and/or second operating position.

## Revendications

1. Dispositif (1) pour chauffer ou stériliser un article de puériculture (1'), comprenant une unité de génération de vapeur (2), laquelle est reliée à une unité destinée à contenir de l'eau (3) en forme de cuve, l'unité destinée à contenir de l'eau (3), dans une première position de fonctionnement, étant reliée à une unité de support (8) présentant une surface de contact au sol (9) pour l'article de puériculture, **caractérisé en ce que**, dans une deuxième position de fonctionnement, l'unité destinée à contenir de l'eau est reliée en variante à une plaque de recouvrement (17) pourvue d'une sortie de vapeur tubulaire (17'), la plaque de recouvrement (17), dans la deuxième position de fonctionnement, étant reliée à un contenant de stérilisation (18) destiné à recevoir l'article de puériculture, et la sortie de vapeur tubulaire (17') de la plaque de recouvrement (17) étant reliée à une soupape d'entrée de vapeur (22) du contenant de stérilisation (18).

2. Dispositif (1) selon la revendication 1, **caractérisé en ce que** la soupape d'entrée de vapeur (22) du contenant de stérilisation (18) peut être amenée, au moyen de la sortie de vapeur tubulaire (17') de la plaque de recouvrement (17), d'une position de fermeture fermant une ouverture d'entrée de vapeur (23) à une position d'ouverture libérant l'ouverture d'entrée de vapeur (23).

3. Dispositif (1) selon la revendication 2, **caractérisé en ce que** la soupape d'entrée de vapeur (22) du contenant de stérilisation (18) comprend un corps de soupape (24) monté coulissant sur un siège de soupape (25) en forme de tubulure, le corps de soupape (24) étant agencé dans la position d'ouverture par l'agencement de la sortie de vapeur tubulaire (17') de la plaque de recouvrement (17).

4. Dispositif (1) selon la revendication 3, **caractérisé en ce que** le corps de soupape (24) de la soupape d'entrée de vapeur (22) présente sur la face inférieure une surface de butée périphérique (26), laquelle coopère avec une surface de butée (27) correspondante sur la face supérieure de la sortie de vapeur tubulaire (17').

5. Dispositif (1) selon la revendication 3 ou 4, **caractérisé en ce que** le siège de soupape (25) en forme de tubulure de la soupape d'entrée de vapeur (22) fait saillie vers l'intérieur sensiblement verticalement depuis une partie fond (18') du contenant de stérilisation (18), la sortie de vapeur tubulaire (17') se dressant sensiblement verticalement depuis le plan principal de la plaque de recouvrement (17).

6. Dispositif (1) selon l'une quelconque des revendications 3 à 5, **caractérisé en ce que** le diamètre intérieur du siège de soupape (25) de la soupape d'entrée de vapeur (22) correspond sensiblement au diamètre extérieur de la sortie de vapeur tubulaire (17').

7. Dispositif (1) selon l'une quelconque des revendications 3 à 6, **caractérisé en ce que** le corps de soupape (24) de la soupape d'entrée de vapeur (17) présente sur la face supérieure un élément de préhension (28) pour le coulissement manuel du corps de soupape (24) entre la position d'ouverture et la position de fermeture.

8. Dispositif (1) selon l'une quelconque des revendications 3 à 7, **caractérisé en ce que** la sortie de vapeur tubulaire (17') de la plaque de recouvrement (17) présente sur la face supérieure une bride de réglage (29), le corps de soupape (24) de la soupape d'entrée de vapeur (22), dans la deuxième position de fonctionnement, étant agencé entre la bride de réglage (29) de la sortie de vapeur tubulaire (17') et le siège de soupape (25) de la soupape d'entrée de vapeur (22).

9. Dispositif (1) selon la revendication 1, **caractérisé en ce que** la plaque de recouvrement (17) est reliée à une poignée destinée à retirer la plaque de recouvrement (17) de l'unité destinée à contenir de l'eau (3).

10. Dispositif (1) selon l'une quelconque des revendications 1 à 9, **caractérisé en ce que** l'unité de support (8) présente au moins une ouverture de passage (12) destinée à soumettre l'article de puériculture à l'effet d'une vapeur d'eau provenant de l'unité destinée à contenir de l'eau (3).

11. Dispositif (1) selon l'une quelconque des revendications 1 à 10, **caractérisé en ce qu'**un logement (7) recevant l'unité destinée à contenir de l'eau (3) est prévu, lequel présente une section de retenue (23) pour la liaison sensiblement étanche à la vapeur sélectivement à l'unité de support (8) ou à la plaque de recouvrement (17).

12. Dispositif (1) selon la revendication 11, **caractérisé en ce que** l'unité de support (8) et/ou la plaque de recouvrement (17) présentent chacune un élément de liaison (30) pour la liaison par enfichage et rotation à une ouverture de réception sur la section de retenue (23) du logement (7).

13. Dispositif (1) selon la revendication 11 ou 12, **caractérisé en ce que** l'unité de support (8) et/ou la plaque de recouvrement (17) présentent chacune un élément de mise en place (14) pour la mise en place sur la section de retenue (23) du logement (7), l'élément de mise en place (14) présentant une bride d'appui horizontale (15, 15') pour la mise en place sur une surface d'appui correspondante de la section de retenue (23) du logement (7) de l'unité destinée à contenir de l'eau (3).

14. Dispositif (1) selon l'une quelconque des revendications 11 à 13, **caractérisé en ce que**, dans la première et/ou la deuxième position de fonctionnement, un élément d'étanchéité est agencé entre l'unité de support (8) ou la plaque de recouvrement (17) et la section de retenue (23) du logement (7).

15. Dispositif (1) selon la revendication 14, **caractérisé en ce qu'**une bride d'étanchéité est prévue en tant qu'élément d'étanchéité sur la section de retenue (23) du logement (7), contre laquelle, dans la première et/ou la deuxième position de fonctionnement, s'applique une bride d'appui (16, 16') correspondante de l'unité de support (8) ou de la plaque de recouvrement (17).
